# FASCICULE DE BREVET EUROPEEN

(11) **EP 0 336 841 B1**
(45) Date de publication et mention de la délivrance du brevet: **26.05.1993**
(21) Numéro de dépôt: 89400935.6
(22) Date de dépôt: 05.04.1989
(51) Int. Cl.: C07D 305/14

(54) **Procédé de préparation de dérivés de la baccatine III et de la désacétyl-10 baccatine III**
Verfahren zur Herstellung von Baccatin-III-Derivaten und von Desacetyl-10-Baccatin-III
Process for the preparation of baccatin III derivatives and of desacetyl-10 baccatin III

(30) Priorité: 06.04.1988 FR 8804513
(43) Date de publication de la demande: 11.10.1989
(73) Titulaire: RHONE-POULENC SANTE, 92160 Antony (FR)
(72) Inventeur: Colin, Michel, F-78770 Thoiry (FR); Guenard, Daniel, F-92120 Montrouge (FR); Gueritte-Voegelein, Françoise, F-91940 Les Ulis (FR); Potier, Pierre, F-75007 Paris (FR)
(74) Mandataire: Pilard, Jacques

(56) Documents cités:
- EP-A- 0 253 738
- J Org. Chem., 1986, no. 51, p.46-50

## Description

La présente invention concerne un procédé de préparation de dérivés du taxane de formule générale : 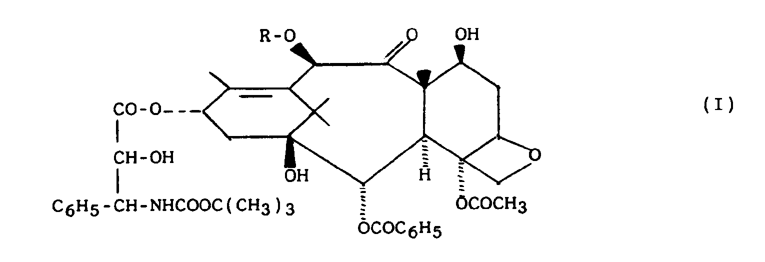
dans laquelle R représente un atome d'hydrogène ou un radical acétyle et ayant la configuration 2'R, 3'S.

Dans la demande de brevet européen EP 253 738 ont été décrits les produits de formule générale (I) et leur préparation. Les produits de formule générale (I), en particulier le produit de formule générale (I) dans laquelle R représente un atome d'hydrogène, présentent des propriétés antitumorales et antileucémiques particulièrement intéressantes.

Selon la demande de brevet européen EP 253 738, les produits de formule générale (I) sont obtenus par action du sel de sodium du N-chlorocarbamate de tertiobutyle sur un produit de formule générale: 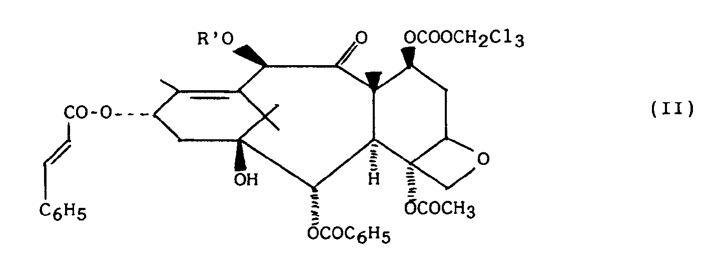
dans laquelle R' représente un radical acétyle ou trichloro-2,2,2 éthoxycarbonyle suivie du remplacement du ou des groupements trichloro-2,2,2 éthoxycarbonyle par un atome d'hydrogène. Ce procédé conduit à un mélange d'isomères qu'il est nécessaire de séparer et il en résulte que la totalité de la baccatine III ou de la désacétyl-10 baccatine III mise en oeuvre pour la préparation du produit de formule générale (II) ne peut être transformée en produit de formule générale (I).

L'article publié dans J. Org. Chem., 51(1) 46-50 (1986) décrit la préparation de la chaîne latérale du taxol de formule : 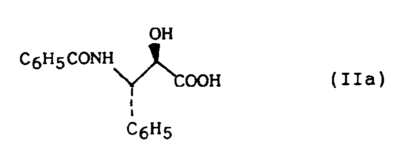
éventuellement sous forme d'ester et suggère seulement son utilisation pour effectuer une synthèse partielle du taxol à partir de la désacétyl-10 baccatine III sans pour autant donner des conditions permettant de réaliser cette synthèse partielle.

Il a maintenant été trouvé, et c'est ce qui fait l'objet de la présente invention, que les produits de formule générale (I) peuvent être obtenus par condensation de l'isomère thréo d'un acide de formule générale : 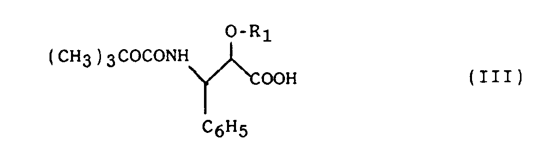
dans laquelle R₁ représente un groupement protecteur de la fonction hydroxy choisi parmi les radicaux méthoxyméthyle, éthoxy-1- éthyle, benzyloxyméthyle, (β-triméthylsilyléthoxy)méthyle, tétrahydropyrannyle et thrichloro-2,2,2 éthoxycarbonyle, sur un dérivé du taxane de formule générale : 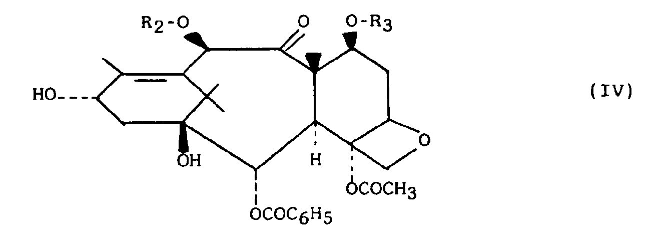
dans laquelle R₂ représente un radical acétyle ou un groupement protecteur de la fonction hydroxy et R₃ représente un groupement protecteur de la fonction hydroxy, le groupement protecteur étant choisi parmi les radicaux trichloro-2,2,2 éthoxycarbonyle et trialkylsilyle dont chaque partie alkyle contient 1 à 3 atomes de carbone, pour obtenir un dérivé du taxane de formule générale : 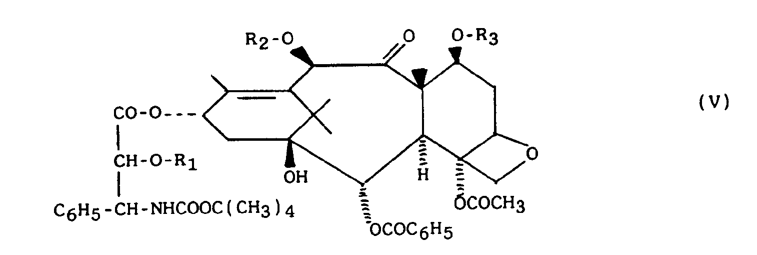
dans laquelle R₁, R₂ et R₃ sont définis comme précédemment, suivie du remplacement des groupements protecteurs R₁, R₃ et éventuellement R₂ par un atome d'hydrogène.

Dans la formule générale (III), le radical R₁ représente, de préférence, le radical éthoxy-1 éthyle.

Généralement, l'estérification du dérivé du taxane de formule générale (IV) par l'acide de formule générale (III) est effectuée en présence d'un agent de condensation tel qu'un carbodiimide comme le dicyclohexylcarbodiimide ou un carbonate réactif comme le dipyridyl-2 carbonate et d'un agent d'activation tel qu'une dialcoylaminopyridine comme la diméthylamino-4 pyridine en opérant dans un solvant aromatique tel que le benzène, le toluène, les xylènes, l'éthylbenzène, l'isopropylbenzène ou le chlorobenzène à une température comprise entre 60 et 90°C.

Il est particulièrement avantageux d'utiliser un excès molaire d'acide de formule générale (III) par rapport au dérivé du taxane de formule générale (IV), l'agent de condensation étant utilisé en quantité stoechiométrique par rapport à l'acide de formule générale (III) et la diméthylamino-4 pyridine étant utilisée en quantité stoechiométrique par rapport au dérivé du taxane de formule générale (IV). Généralement, on utilise au moins 4 moles d'acide de formule générale (III) par mole de dérivé du taxane de formule générale (IV).

L'élimination des groupements protecteurs de l'ester obtenu de formule générale : 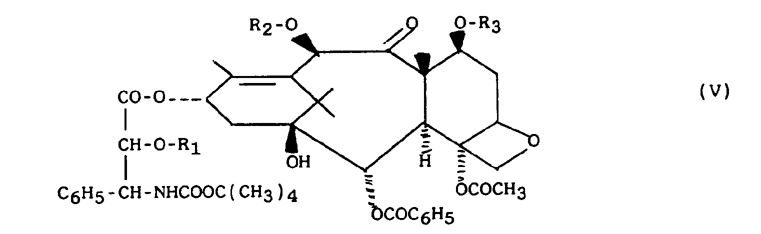
peut être effectuée au moyen de zinc en présence d'acide acétique à une température comprise entre 30 et 60°C ou par traitement au moyen d'un acide minéral ou organique tel que l'acide chlorhydrique ou l'acide acétique en solution dans un alcool aliphatique contenant 1 à 3 atomes de carbone en présence de zinc.

L'acide de formule générale (III) peut être obtenu par saponification d'un ester de formule générale : 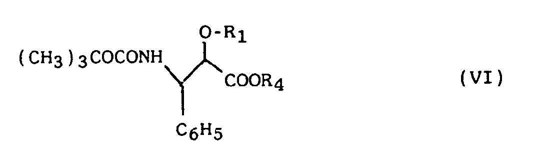
dans laquelle R₁ est défini comme précédemment et R₄ représente un radical alcoyle contenant 1 à 4 atomes de carbone et, de préférence, un radical éthyle, au moyen d'une base minérale telle qu'un hydroxyde de métal alcalin (lithine, soude), un carbonate ou bicarbonate de métal alcalin (bicarbonate de sodium, carbonate de potassium) en milieu hydro-alcoolique tel qu'un mélange méthanol-eau en opérant à une température comprise entre 10 et 40°C, de préférence voisine de 25°C.

Le produit de formule générale (VI) peut être obtenu dans les conditions habituelles de préparation des éthers, et plus particulièrement selon les procédés décrits par J.N. Denis et coll., J. Org. Chem., 51, 46-50 (1986), à partir du produit de formule générale : 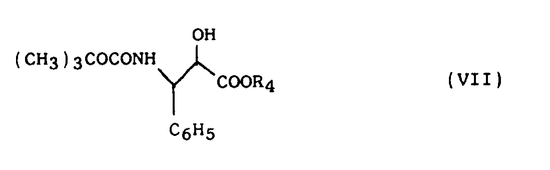
dans laquelle R₄ est défini comme précédemment.

Le produit de formule générale (VII) peut être obtenu par action du dicarbonate de di-tertiobutyle sur un produit de formule générale : 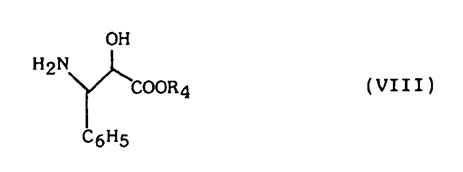
dans laquelle R₄ est défini comme précédemment. Généralement, on opère dans un solvant organique tel que le chlorure de méthylène en présence d'une base minérale telle que le bicarbonate de sodium.

Le produit de formule générale (VIII) peut être obtenu par réduction d'un azide de formule générale : 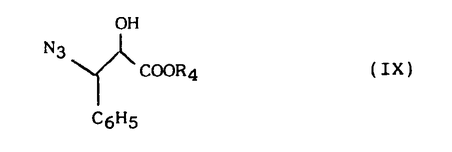
xdans laquelle R₄ est défini comme précédemment, qui est obtenu selon les méthodes connues d'ouverture d'un époxyde de formule générale : 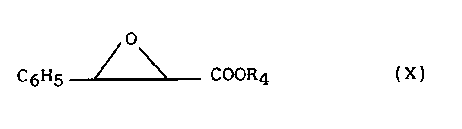
dans laquelle R₄ est défini comme précédemment, au moyen d'azoture de sodium dans l'éthanol à chaud.

L'époxyde de formule générale (X) peut être obtenu dans les conditions décrites par F.W. Bachelor et R.K. Bansal, J. Org. Chem., 34, 3600-04 (1969).

Pour la mise en oeuvre du procédé selon l'invention, il est particulièrement avantageux d'utiliser les produits de formules générales (VI) à (X) dans lesquelles R₄ représente un radical éthyle.

Lorsque l'on utilise un produit de formule générale (X) dans laquelle R₄ représente un radical autre que éthyle, par exemple un radical tertiobutyle, il est nécessaire, après ouverture de l'époxyde de formule générale (X), d'effectuer une réaction de transestérification pour transformer le radical R₄ en radical éthyle.

Le dérivé du taxane de formule générale (IV) dans laquelle R₂ représente un radical acétyle ou trichloro-2,2,2 éthyle peut être obtenu par action du chloroformiate de trichloro-2,2,2 éthyle sur la baccatine III ou la désacétyl-10 baccatine III en opérant dans un solvant organique basique tel que la pyridine à une température comprise entre 0 et 50°C.

Le dérivé du taxane de formule générale (IV) dans laquelle R₂ représente un radical acétyle et R₃ représente un radical trialkylsilyle peut être obtenu par action d'un halogénotrialkylsilane sur la baccatine III ou la désacétyl-10 baccatine III, suivie, dans ce dernier cas, de l'acétylation de la trialkylsilyl-7 désacétyl-10 baccatine III intermédiairement obtenue.

Généralement la réaction de l'halogénotrialkylsilane sur la baccatine III ou la désacétyl-10 baccatine III s'effectue à une température voisine de 20°C en opérant dans un solvant organique basique tel que la pyridine ou dans un solvant organique inerte tel que le chloroforme ou le dichlorométhane en présence d'une amine tertiaire telle que la triéthylamine, la pyridine ou la base de Hunig.

L'acétylation de la trialkylsilyl-7 désacétyl-10 baccatine III est généralement effectuée au moyen de chlorure d'acétyle en opérant à une température voisine de 0°C dans un solvant organique basique tel que la pyridine ou dans un solvant organique inerte tel que le chloroforme, le chlorure de méthylène ou le dichloroéthane en présence d'une amine tertiaire telle que la pyridine ou la base de Hunig.

L'exemple suivant, donné à titre non limitatif, montre comment l'invention peut être mise en pratique.

### EXEMPLE

Dans un ballon tricol de 500 cm3 muni d'une agitation et d'un thermomètre, on introduit, sous atmosphère d'argon, 7,9 g d'acide (éthoxy-1 éthoxy)-2 tertiobutyloxycarbonylamino-3 phényl-3 propionique, thréo (22,4 mmoles), 150 cm3 de toluène anhydre et 4,6 g de dicyclohexylcarbodiimide (22,4 mmoles), 5 g de produit de formule générale (IV) dans laquelle R₂ et R₃ représentent chacun un radical trichloro-2,2,2 éthoxycarbonyle (5,6 mmoles) et 0,68 g de diméthylamino-4 pyridine (5,6 mmoles). Le mélange est chauffé pendant 7 heures à 70°C sous atmosphère d'argon. Après refroidissement à 20°C, le précipité formé est séparé par filtration puis lavé par 50 cm3 de toluène froid.

Le filtrat est concentré à sec puis il est repris par 150 cm3 de chlorure de méthylène. La solution chlorométhylénique est lavée 2 fois par 50 cm3 d'eau. La phase organique est concentrée à sec.

On obtient ainsi 13,5 g d'un produit qui est chromatographié sur 270 g de silice Géduran en éluant avec un mélange chlorure de méthylène-méthanol (98-2 en volumes). Les impuretés sont éliminées en éluant avec 1 litre du mélange, puis en éluant à nouveau avec 1 litre du mélange, on obtient 8 g d'ester de formule générale (V) dans laquelle R₁ représente un radical (éthoxy-1 éthyl) et R₂ et R₃ représentent chacun un radical trichloro-2,2,2 éthoxycarbonyle. En poursuivant l'élution avec 1 litre du même mélange, on récupère 3,2 g de dérivé du taxane de départ qui peut être recristallisé dans le toluène.

On dissout 8 g d'ester obtenu ci-dessus dans 200 cm3 d'un mélange acide acétique-méthanol (1-1 en volumes), puis on ajoute 8 g de poudre de zinc fraîchement réactivé. Après 1 heure à 60°C sous atmosphére d'argon, le mélange réactionnel est refroidi à 20°C puis est filtré. Le produit solide est rincé par 50 cm3 du mélange acide acétique-méthanol. Les filtrats réunis sont concentrés à sec, puis le résidu est repris par l'acétate d'éthyle. Un insoluble est séparé par filtration et lavé 3 fois avec 60 cm3 au total d'acétate d'éthyle.

Les phases organiques réunies sont lavées avec 100 cm3 d'une solution à demi-saturée de bicarbonate de sodium, puis avec 50 cm3 d'eau. Les phases organiques sont séchées sur sulfate de sodium. Après filtration et évaporation du solvant, on obtient un résidu (5,5 g) qui est chromatographié sur 162 g de silice Géduran en éluant avec un mélange hexane-acétate d'éthyle (1-1 en volumes). On sépare 2,4 g d'impuretés, puis 0,595 g de dérivé oxy-aminé (2'R, 3'S) puis 1,794 g de produit de formule (I) (2'R, 3'S) dans laquelle R représente un atome d'hydrogène dont la pureté est de 90 %.

Le produit est identique à celui qui est décrit dans l'exemple 1 de la demande de brevet européen EP 253 738.

L'acide (éthoxy-1 éthoxy)-2 t.butoxycarbonylamino-3 phényl-3 propionique, thréo peut être préparé de la manière suivante :

On dissout 10 g d'(éthoxy-1 éthoxy)-2 t.butoxycarbonylamino-3 phényl-3 propionate d'éthyle, thréo dans 500 cm3 d'éthanol. On ajoute 3,3 g de lithine, 1H₂O en solution dans 250 cm3 d'eau. La solution trouble est agitée pendant 15 heures à une température voisine de 20°C. On évapore l'éthanol sous pression réduite. On ajoute 250 cm3 d'eau puis on lave la phase aqueuse avec au total 250 cm3 de chlorure de méthylène. La phase aqueuse est acidifiée par addition d'acide chlorhydrique 1N jusqu'à pH = 3, en extrayant au fur et à mesure par 750 cm3 au total de chlorure de méthylène. Après séchage et concentration à sec, on obtient, avec un rendement de 95 %, 8,8 g d'acide (éthoxy-1 éthoxy)-2 t.butoxycarbonylamino-3 phényl-3 propionique, thréo qui, après recristallisation dans l'acétate d'éthyle, présente les caractéristiques suivantes :
- point de fusion : 152-154°C
- spectre infra-rouge (en solution dans le chloroforme) : 3450, 2990, 2940, 1760 et 1735 cm⁻¹.

L'(éthoxy-1 éthoxy)-2 t.butoxycarbonylamino-3 phényl-3 propionate d'éthyle, thréo peut être préparé de la manière suivante :

Dans un ballon de 2 litres muni d'une agitation et d'un thermomètre, on introduit, sous atmosphère d'argon, 30 g d'hydroxy-2 t.butoxycarbonylamino-3 phényl-3 propionate d'éthyle, thréo en solution dans 1000 cm3 de chlorure de méthylène, 2,4 g de p.toluènesulfonate de pyridinium et 93 cm3 de vinyléthyléther. Après 6 heures à une température voisine de 20°C, on ajoute quelques gouttes de pyridine de façon à amener le pH à 7. La solution organique est lavée avec 200 cm3 d'eau à demi saturée de chlorure de sodium puis séchée sur sulfate de magnésium. Après filtration et élimination des solvants sous pression réduite, on obtient, avec un rendement voisin de 100 %, 38,6 g d'(éthoxy-1 éthoxy)-2 t.butoxycarbonylamino-3 phényl-3 propionate d'éthyle, thréo dont la structure est confirmée par le spectre de résonance magnétique nucléaire du proton et par le spectre de masse.

L'hydroxy-2 t.butoxycarbonylamino-3 phényl-3 propionate d'éthyle, thréo peut être préparé de la manière suivante :

Dans un ballon tricol de 4 litres muni d'une agitation, d'un thermomètre et d'un réfrigérant, on introduit 136 g d'hydroxy-2 amino-3 phényl-3 propionate d'éthyle, thréo en solution dans 1500 cm3 de chlorure de méthylène puis lentement 196 g de dicarbonate de di t.butyle en solution dans 500 cm3 de chlorure de méthylène. Il y a dégagement de gaz carbonique et élévation de la température. Après 20 minutes de réaction, on ajoute 50 g de bicarbonate de sodium puis laisse la température descendre au voisinage de 20°C en 3 heures tout en agitant. Après filtration, la phase organique est lavée 2 fois à l'eau puis séchée sur sulfate de magnésium. Après filtration et évaporation des solvants, on obtient une huile qui prend en masse (305 g). Le solide est repris par 3500 cm3 d'hexane. Après 15 heures à une température de 4°C, les cristaux obtenus sont séparés par filtration et lavés à l'hexane. On obtient ainsi, avec un rendement de 73 %, 148 g d'hydroxy-2 t.butoxycarbonylamino-3 propionate d'éthyle, thréo dont la structure est confirmée par le spectre de résonance magnétique nucléaire du proton et par le spectre de masse.

L'hydroxy-2 amino-3 phényl-3 propionate d'éthyle peut être préparé de la manière suivante :

Dans un ballon tricol de 4 litres, on introduit 178 g d'hydroxy-2 azido-3 phényl-3 propionate d'éthyle, thréo en solution dans 2 litres d'éthanol à 95°C, puis on ajoute 20 g de palladium sur charbon à 10 % de palladium (p/p). Après une purge à l'argon, on fait passer un courant d'hydrogène dont le débit est réglé de façon à maintenir la température inférieure à 30°C. Après 1 heure, le ballon est purgé à l'argon. Le mélange réactionnel est filtré sur célite puis est rincé avec de l'éthanol. Après concentration à sec, on obtient une huile qui cristallise pour donner, avec un rendement de 92 %, 146 g d'hydroxy-2 amino-3 phényl-3 propionate d'éthyle, thréo dont la structure est confirmée par le spectre de résonance magnétique nucléaire du proton et le spectre de masse.

L'hydroxy-2 azido-3 phényl-3 propionate d'éthyle, thréo peut être préparé de la manière suivante :

Dans un ballon tricol de 4 litres, on dissout 194 g d'hydroxy-2 azido-3 phényl-3 propionate de t.butyle thréo dans 1 litre d'éthanol absolu. On ajoute 550 cm3 d'une solution fraîchement préparée d'acide chlorhydrique dans l'éthanol à 13 % en poids. Après 3 heures à une température voisine de 20°C, l'éthanol est évaporé sous pression réduite. Le résidu est repris par 1,5 litre de chlorure de méthylène. La solution chlorométhylénique est lavée avec 200 cm3 d'une solution saturée de bicarbonate de sodium puis à l'eau. Aprés séchage et évaporation du solvant, on obtient, avec un rendement de 99,7 %, 180 g d'hydroxy-2 azido-3 phényl-3 propionate d'éthyle thréo dont la structure est confirmée par le spectre de résonance magnétique nucléaire du proton et par le spectre de masse.

L'hydroxy-2 azido-3 phényl-3 propionate de t.butyle thréo peut être préparé de la manière suivante :

Dans un tricol de 6 litres muni d'une agitation, d'un thermomètre et d'un réfrigérant, on introduit 189 g de phényl-3 glycidate de t.butyle en solution dans 3 litres d'éthanol. On ajoute 95 g d'azoture de sodium et 75 g de chlorure d'ammonium puis chauffe à 75°C pendant 20 heures. On distille les 2/3 de l'éthanol sous pression réduite à 50°C puis on ajoute 4 litres d'eau et enfin termine l'évaporation de l'éthanol. Après refroidissement de la suspension aqueuse, on obtient un précipité cristallin qui est séparé par filtration et lavé à l'eau. Après séchage sous pression réduite en présence d'anhydride phosphorique, on obtient, avec un rendement de 82,4 %, 186,4 g d'hydroxy-2 azido-3 phényl-3 propionate de t.butyle thréo dont la structure est confirmée par le spectre de résonance magnétique nucléaire du proton et par le spectre de masse.

Le phényl-3 glycidate de t.butyle peut être préparé selon le procédé décrit par F.W. Bachelor et R.K. Bansal, J. Org. Chem., 34, 3600 (1969). Le produit obtenu est purifié par chromatographie sur silice Merck 7734® afin d'obtenir 200 g de phényl-3 glycidate de t.butyle cis.

Le produit de formule générale (IV) dans laquelle R₂ et R₃ représentent chacun un radical trichloro-2,2,2 éthoxycarbonyle peut être préparé selon le procédé décrit dans la demande de brevet européen EP 253 738.

## Revendications

1. Procédé de préparation d'un dérivé du taxane de formule générale : 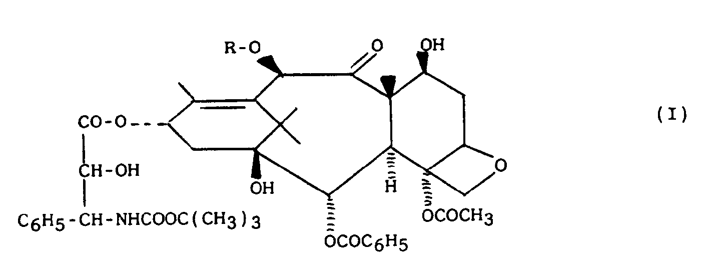 dans laquelle R représente un atome d'hydrogène ou un radical acétyle, et ayant la configuration 2'R,3'S, caractérisé en ce que l'on condense l'isomère thréo d'un acide de formule générale : 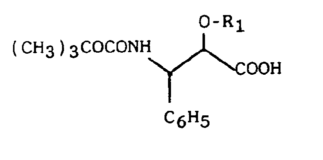 dans laquelle R₁ représente un groupement protecteur de la fonction alcool choisi parmi les radicaux méthoxyméthyle, éthoxy-1 éthyle, benzyloxyméthyle, (β-triméthylsilyléthoxy) méthyle, tétrahydropyrannyle ou trichloro-2,2,2 éthoxycarbonylé, sur un dérivé du taxane de formule générale : 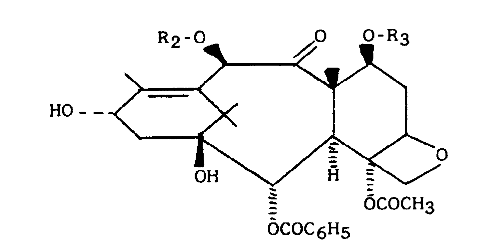 dans laquelle R₂ représente un radical acétyle ou un groupement protecteur de la fonction hydroxy et R₃ représente un groupement protecteur de la fonction hydroxy, le groupement protecteur étant choisi parmi les radicaux trichloro-2,2,2 éthoxycarbonyle et trialkylsilyle dont chaque partie alkyle contient 1 à 3 atomes de carbone en présence d'un agent de condensation choisi parmi les carbodiimides et les carbonates réactifs et d'un agent d'activation choisi parmi les dialcoylaminopyridines en opérant dans un solvant aromatique choisi parmi le benzène, le toluène, les xylènes, l'éthylbenzène, l'isopropylbenzène et le chlorobenzène à une température comprise entre 60 et 90°C, pour obtenir un dérivé du taxane de formule générale : 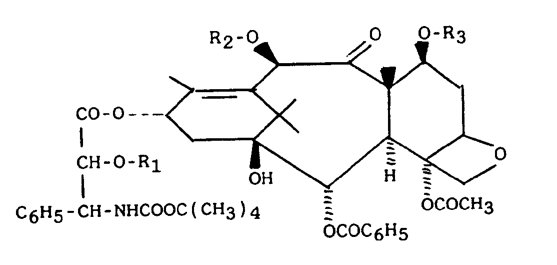 dans laquelle R₁, R₂ et R₃ sont définis comme précédemment, dont on remplace les groupements protecteurs R₁, R₃ et éventuellement R₂ par des atomes d'hydrogène au moyen de zinc en présence d'acide acétique ou au moyen d'un acide minéral ou organique en solution dans un alcool aliphatique contenant 1 à 3 atomes de carbone, puis sépare le produit de formule générale (I) sous forme 2'R,3'S.

## Claims

1. Process for the preparation of a taxane dérivative of general formula: 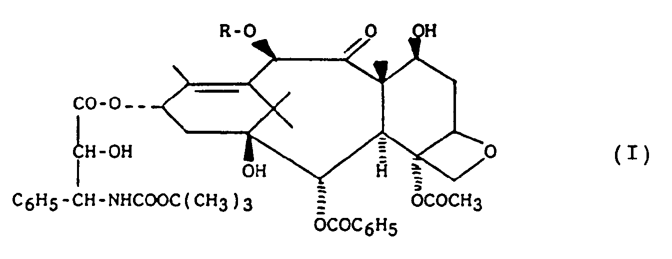 in which R represents a hydrogen atom or an acetyl radical, and having the configuration 2'R,3'S, characterised in that the threo isomer of an acid of general formula: 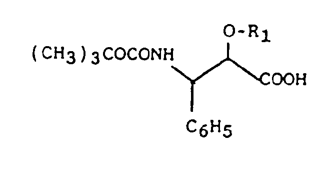 in which R₁ represents a protective group for the alcohol functional group, chosen from methoxymethyl, 1-ethoxyethyl, benzyloxymethyl, (β-trimethylsilylethoxy)methyl, tetrahydropyranyl or 2,2,2-trichloroethoxycarbonyl radicals, is subjected to a condensation reaction with a taxane derivative of general formula: 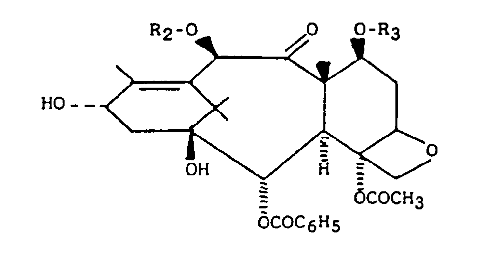 in which R₂ represents an acetyl radical or a protective group for the hydroxyl functional group and R₃ represents a protective group for the hydroxyl functional group, the protective group being chosen from 2,2,2-trichloroethoxycarbonyl and trialkylsilyl radicals, each alkyl part of said trialkylsilyl radicals containing 1 to 3 carbon atoms, in the presence of a condensing agent chosen from carbodiimides and reactive carbonates and an activating agent chosen from dialkylaminopyridines, the reaction being carried out in an aromatic solvent chosen from benzene, toluene, xylenes, ethylbenzene, isopropylbenzene and chlorobenzene, at a temperature of between 60 and 90°C, in order to obtain a taxane derivative of general formula: 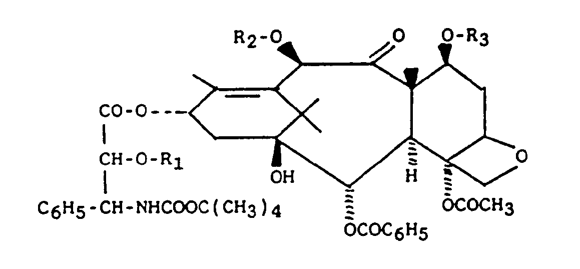 in which R₁, R₂ an R₃ are defined as above, in which the protective groups R₁, R₃ and, where appopriate, R₂ are replaced by hydrogen atoms by means of zinc in the presence of acetic acid or by means of an inorganic or organic acid in solution in an aliphatic alcohol containing 1 to 3 carbon atoms, and the product of general formula (I) in the 2'R, 3'S form is then separated off.

## Patentansprüche

1. Verfahren zur Herstellung eines Taxanderivats der allgemeinen Formel 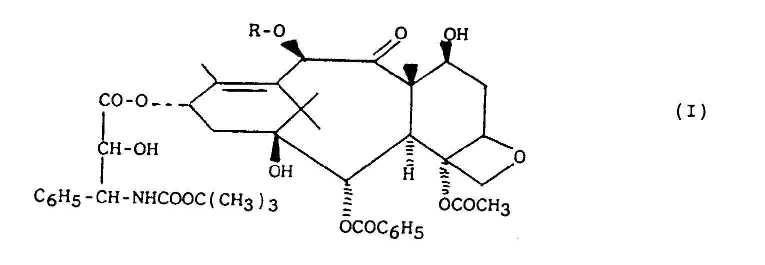 worin R für ein Wasserstoffatom oder einen Acetylrest steht, das die 2'R,3'S-Konfiguration besitzt, dadurch gekennzeichnet, daß man das threo-Isomere einer Säure der allgemeinen Formel 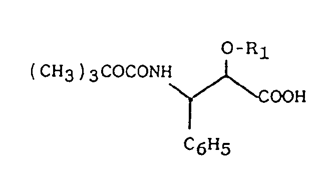 worin R₁ eine Schutzgruppe für die Alkoholfunktion, ausgewählt unter den Methoxymethyl-, 1-Ethoxyethyl-, Benzyloxymethyl-, (β-Trimethylsilylethoxy)-methyl-, Tetrahydropyranyl-oder 2,2,2-Trichlonethoxycarbonylresten, bedeutet, mit einem Taxanderivat der allgemeinen Formel 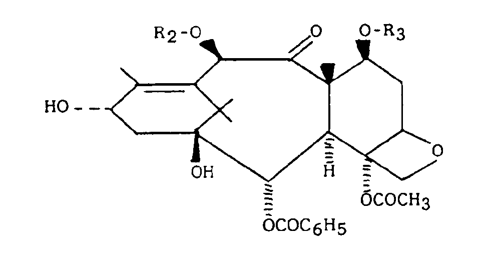 worin R₂ für einen Acetylrest oder eine Schutzgruppe für die Hydroxyfunktion steht, und R₃ eine Schutzgruppe für die Hydroxyfunktion bedeutet, wobei die Schutzgruppe unter den 2,2,2-Trichlorethoxycarbonyl- und Trialkylsilylresten, deren Alkylteil 1 bis 3 Kohlenstoffatome aufweist, ausgewählt ist, in Gegenwart eines Kondensationsmittels, ausgewählt unter den Carbodiimiden und den reaktiven Carbonaten, und eines Aktivierungsmittels, ausgewählt unter den Dialkylaminopyrridinen, kondensiert, wobei man in einem aromatischen Lösungsmittel, ausgewählt unter Benzol, Toluol, den Xylolen, Ethylbenzol, Isopropylbenzol und Chlorbenzol, bei einer Temperatur zwischen 60 und 90°C arbeitet, um zu einem Taxanderivat der allgemeinen Formel 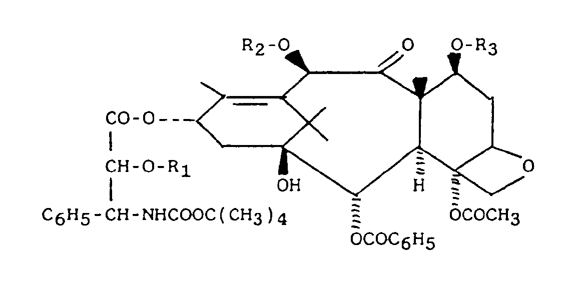 zu gelangen, worin R₁, R₂ und R₃ die vorstehend angegebene Bedeutung besitzen, dessen Schutzgruppen R₁, R₃ und gegebenenfalls R₂ man durch Wasserstoff mit Hilfe von Zink in Anwesenheit von Essigsäure oder mit Hilfe einer Mineral- oder organischen Säure in Lösung in einem aliphatischen Alkohol mit 1 bis 3 Kohlenstoffatomen austauscht, wonach man das Produkt der allgemeinen Formel (I) in der 2'R,3'S-Form abtrennt.
